# EUROPEAN PATENT APPLICATION

(11) **EP 3 669 757 A1**
(43) Date of publication of application: **24.06.2020**
(21) Application number: 18213291.0
(22) Date of filing: 18.12.2018
(51) Int. Cl.: A61B 5/00, A61B 5/02, A61B 5/024, A61B 5/11

(54) **SYSTEM AND METHOD FOR DETECTING FLUID ACCUMULATION**

(71) Applicant: Koninklijke Philips N.V., 5656 AG Eindhoven (NL)
(72) Inventor: BULUT, Murtaza, 5656 AE Eindhoven (NL); VAN LIESHOUT, Ron Martinus Laurentius, 5656 AE Eindhoven (NL); DELLIMORE, Kiran Hamilton J., 5656 AE Eindhoven (NL); JOSHI, Rohan, 5656 AE Eindhoven (NL)
(74) Representative: de Haan, Poul Erik

(57) **Abstract**

A system is provided for detecting fluid accumulation in a subject. A movement induced by the beating of the heart is analyzed over time, by monitoring a movement, pressure or force. Changes in density caused by fluid accumulation result in changes in the sensor arrangement signals. Changes are used to indicate that fluid accumulation such as internal bleeding is likely to have taken place.

## Description

### FIELD OF THE INVENTION

This invention relates to a system and method for detecting fluid accumulation in the body, for example internal bleeding caused by hemorrhage.

### BACKGROUND OF THE INVENTION

Hemorrhage is a major problem in postoperative care, emergency departments, acute care settings and in stroke patients. In particular, timely and confident monitoring and detection of internal hemorrhage at the bedside remains an important challenge across multiple settings.

When a healthy person loses more than 1000ml of blood, the body will respond by increasing the heart rate (HR) and there will be consequent changes in blood pressure. However, patients on medications (e.g., beta blockers) and those suffering from neuropathy (e.g. due to diabetes) may have a compromised ability to compensate for blood loss and might present with only limited changes in vital signs. Moreover, younger patients have a larger compensation mechanism for blood volume fluctuation and thus the vital sign changes associated with blood loss will occur later.

When a sustained change of vital signs is clinically observed, the confirmation of internal hemorrhage is typically carried out by performing a scan using X-ray, CT or ultrasound. Symptoms and sequelae of hemorrhage relate to perfusion of tissues.

A loss of 15% or less of blood volume may not be associated with any change in vital signs. As a result, a hemorrhage remains undiagnosed until a significant quantity of blood loss has taken place.

For example, compensated hemorrhagic shock corresponds to a blood loss of less than 1000ml, below which the heart rate, blood pressure, respiration and capillary refill remain normal. Mild shock corresponds to a blood loss of 1000-1500ml but only small changes in physiological parameters are observable. Moderate shock corresponds to a blood loss of 1500-2000ml. There is then a marked fall in blood pressure, increased heart rate (>120 bpm), a delayed capillary refill, and moderate tachypnea. At this stage the subject is typically confused.

Severe shock corresponds to a blood loss of over 2000ml. There is then a profound fall in blood pressure, greatly increased heart rate (>140 bpm), a delayed capillary refill, and marked tachypnea (respiratory collapse). The subject is then lethargic and obtunded.

Known bedside warning systems for diagnosis rely on measures of heart rate and blood pressure, but these vitals are not specific for internal hemorrhage and may even be insensitive for certain patient groups (e.g., those on beta blockers). Because the elevation of heart rate occurs only after more than 1000ml of blood loss, it is a late marker. In addition, heart rate elevation can be caused by numerous other factors (e.g. stress, medication) and it therefore is not specific to internal hemorrhage.

As explained above, advanced imaging methods for definitive radiological confirmation, such as CT scans, may be used to confirm a diagnosis if hemorrhage is suspected. This further adds to a delay in detection. Furthermore, these methods are expensive, time consuming (since the clinical workflow is complex), burdensome on the patient and difficult to fit in the workflow. They also do not provide continuous measurement.

It is also worth mentioning that transporting certain patients, such as those who have undergone major surgery, is not without risk, further increasing the need for improved confidence in diagnosing internal hemorrhage at the bedside.

Sela, I et. al., "Measuring Left Ventricular Ejection Time using Under-the-Mattress Sensor discloses the measurement of Left Ventricular Ejection Time (LVET) by measuring the cardio-ballistic effect using a piezoelectric sensor under the mattress. LVET shortening is considered to result from loss of blood during internal hemorrhage. The sensor is positioned below the sternum to be as close to the heart as possible.

There remains a need for more specific and sensitive indicators for detecting internal bleeding (also known as hypovolemic shock or hemorrhagic shock) and for detecting fluid accumulation in the body more generally.

### SUMMARY OF THE INVENTION

The invention is defined by the claims.

According to examples in accordance with an aspect of the invention, there is provided a system for detecting fluid accumulation in a body region of a subject, comprising:
a sensor arrangement for obtaining a force, motion or pressure signal, induced by the beating of the heart of the subject, wherein the signal varies in dependence on the density distribution of the body region; and
a processor, which is adapted to analyze changes in the sensor arrangement signals over time thereby to determine that fluid accumulation has taken place based on detecting changes in said density distribution.

This system tracks changes in a sensor arrangement signal and is based on the detection of changes in the distribution of different densities of material caused by fluid accumulation.

The fluid accumulation may for example be the result of internal bleeding, and the density changes result from loss of blood from the circulatory system and the presence of the blood at other locations. In particular, the change in density distribution changes the way pressure pulses caused by the heart propagate through the body (in particular the region of interest) to the sensor arrangement.

The sensor arrangement for example functions in the manner of a ballistocardiography (BCG) sensor. It is known to use a BCG signal for heart rate and respiration detection, and this invention makes use of analysis of a BCG signal, or more generally a sensor arrangement signal based on pressure, movement or force, for fluid accumulation. The invention is based on detecting trends in the signal and with temporal analysis so that naturally occurring changes can be distinguished from fluid accumulation indicating a medical condition, for example caused by internal bleeding. Thus, generally, the invention involves measuring (using a sensor arrangement) a pressure, and/or force and/or movement signal over time, and based on the observed differences over time determining if fluid accumulation has taken place.

The sensor arrangement signal comprises at least a component which has been modulated as a result of the pressure pulsed caused by the beating of the heart having passed through the body region, preferably directly. The signal processing is for detecting changes in the density distribution (in 3D space) of the body region. This does not mean that any density value is provided as output, rather that the signal processing would respond to density changes even if the heart beat characteristics were to remain constant.

The sensor arrangement may be:
for location on an opposite side of the body region to the heart of the subject; or
for sensing from an opposite side of the body region to the heart of the subject; or
for sensing movement of an object coupled to the subject in such a way that movement of the object is induced by the beating of the heart.

In some examples, the region of interest is located between the heart and the sensor so that the heart beat pressure wave propagates through the region of interest to reach the sensor.

In such examples, the sensor is preferably located laterally displaced from the heart, for example by at least 10cm, preferably by at least 20cm and more preferably by at least 30cm. By laterally displaced is meant that the position of the sensor, when projected onto a plane parallel to the general plane of the body and which passes through the heart, is displaced from the position of the heart in that plane. Thus, at least some of the main paths through the body from the heart to the sensor include the body region of interest, which itself is typically not directly above or below the heart (i.e. in a direction perpendicular to the plane).

The same concept may be applied to a non-contact sensor, where there may still be sensing from a particular area. In such a case, the location from which sensing takes place is again laterally displaced from the heart, for example by at least 10cm, preferably by at least 20cm and more preferably by at least 30cm. However, the path does not need to be direct, in that any configuration may be used as long as fluid accumulation has a measurable influence on the sensor signal. The beating heart may even induce movement of another object (such as a bed frame).

The coupling of vibrations to that object may again be at a location of the body of the subject which is laterally displaced from the heart, for example by at least 10cm, preferably by at least 20cm and more preferably by at least 30cm.

Generally, the region of interest is not the heart, so that the aim is to detect fluid accumulation remote from the heart based on the analysis of pressure waves that have propagated through the region of interest, which itself is remote from the heart. Thus, the sensor needs to be arranged so that there is a measurable influence on the propagation of pressure waves between the heart and the sensor, and in particular caused by a change in density distribution of the media through which that pressure wave has propagated.

The invention does not require analysis of the characteristics of an individual heart beat. Instead, it relies on the propagation of the overall (cumulative) heat beat pressure pulse and the way that overall pressure pulse is transmitted through the body. Thus, the signal analysis required is not aimed at measuring a property of the heart, but is aimed at measuring a property of the body.

In order to estimate properties of the heart (such as LVET) a signal is needed from which extraction is able to be performed of parameters related to the heart cycles or heart beats such as beat intervals, or beat frequencies. Thus, the analysis is based on detection of periodic components. The signal analysis thus requires pre-processing of the signal acquired due to the beating heart for emphasizing, or calculating features related to this periodic behavior.

The analysis of the more general pulse propagation in accordance with the invention enables simpler signal processing, in that there is no need to extract a periodic behavior from the captured signal, or to determine parameters specifically relating to the individual heart cycles or the heart beat periodicity.

For example, the analysis may be performed based on sample of data in respect of a duration which includes only a single heart beat (e.g. 0.5s) so that no periodicity information relating to the heart beat is needed. The analysis may be performed based on a sample of data of a longer duration (for example seconds or tens of seconds). However, the analysis still makes no use of information relating to the periodicity of the heart beat, and indeed the frequency components relating to such periodicity may in some examples of possible signal processing be specifically excluded from the signal processing (by high pass or band pass filtering).

The sensor arrangement preferably comprises:
one or more pressure sensors; and/or
one or more inertial sensors.

The sensor arrangement is located on the opposite side of the heart to the region of interest, so that pressure waves created by the heart will pass through the region of interest before sensing. Note that optical sensing is also possible, and indeed optical sensing approaches for BCG sensors have been proposed. The inertial sensors may comprise accelerometers, gyroscopes or other gyro-sensors.

The sensor arrangement may comprise:
a sensor arrangement for wearing on the head e.g. to monitor bleeding on the brain;
a sensor arrangement for wearing around the waist, e.g. to monitor bleeding in the torso;
a sensor arrangement for wearing on the back e.g. to monitor fluid accumulation in the lungs;
a sensor arrangement for wearing on the leg or feet, e.g. to monitor fluid accumulation at any location below the heart;
a sensor arrangement on which the subject is to stand, lie or sit;
a non-contact sensor.

In one example of signal processing approach, the processor may be adapted to:
perform feature extraction to isolate a feature of interest of the sensor arrangement signals;
determine a parameter of the feature of interest of the sensor arrangement signals; and
determine from changes in the determined parameter that fluid accumulation has taken place.

The feature of interest to be extracted may be predefined or preselected, if it is known in advance, or it may be determined dynamically, for example by a machine learning algorithm.

To analyze if the characteristics of the sensor arrangement signal has changed, specific, informed features can be extracted, and changes in these feature values can be assessed.

A more general time-series analysis may be performed by which the variation of signal shapes or other properties is monitored over time. Many features can be extracted from the time domain signal, and using these features machine learning tools can be used to determine if fluid accumulation has happened. This is a machine learning, or data driven approach. This would involve training using sensor arrangement signals which are obtained with and without fluid accumulation. Alternatively, or in addition, the machine learning can be applied in an up-supervised manner where extracted features would be used to evaluate if clusters can be formed.

Deep learning methods do not require features to be calculated ahead. Instead, the raw sensor arrangement signals can be used as an input, and fluid accumulation can be detected. A neural network of the deep learning method would be first trained with annotated sensor arrangement data.

In the feature-based approach, the parameter for example comprises a measure of signal energy, signal strength or signal variance. Thus it relates to the level of signal attenuation (or amplification or variation) between the heart and the sensor arrangement.

The processor is preferably adapted to analyze changes in the sensor arrangement signals over a time frame of at least two minutes.

This time frame relates to the period during which the subject is monitored. This should be long enough to ensure that between the start and end of the monitoring enough fluid has been accumulated. Depending on the intensity of the fluid accumulation (e.g. bleeding) this can generally range from a few minutes to hours. The subject may be monitored continuously in this time frame. The processing of the signals to derive a measure of fluid accumulation for example takes place in a time window which may vary from a fraction of a second (e.g. 0.5s) to a few seconds to a few minutes (e.g. 1 minute).

The time windows for sequential parameter determinations may overlap. Thus, there may be a moving time window over which analysis is carried out. The overlap is for example between 50% (e.g. a 30 second overlap of a 1 minute time window, with new measurements every 30 seconds) and 95% (e.g. 19 second overlap of a 20 second time window, with new measurements each second).

In one set of examples, the processor may comprise a decomposition algorithm for implementing the feature extraction by decomposing the sensor arrangement signals into frequency components, wherein the feature of interest comprises a frequency component in a frequency band which lies above 1.67 Hz or above a determined heart rate frequency of the subject.

The high frequency components (relative to the heart rate) are found to be of particular interest in representing changes in the density distribution caused by internal bleeding or other fluid accumulation.

The decomposition algorithm is for example adapted to implement empirical mode decomposition. This is one suitable approach for the frequency analysis. Other approaches include wavelet transforms, or band-pass filtering (i.e. using filter banks).

In the feature based approach, the parameter may comprise a Hilbert transform or an energy of the extracted frequency based feature and the processor may then comprise a Hilbert transformation unit for implementing the Hilbert transform.

In another set of examples, the parameter comprises an energy or a Hilbert transform of the raw sensor arrangement signals (so without the preceding frequency decomposition), and the system may then comprises a Hilbert transformation unit for implementing the Hilbert transform. Thus, the frequency based decomposition is not essential, and the processing may be carried out on the raw signal. Note that the raw signal may need to be processed before any features or parameters are calculated, for example a transformation or filtering of the raw signal before analysis. The term "raw signal" should be understood accordingly, as a signal which is not decomposed into frequency components, but by have had some signal pre-processing. This may be of interest when the acquired raw signal is noisy, or contains artefacts and therefore needs to be cleaned before any further analysis.

This processing of the raw signal may also be applied before signal decomposition is applied.

The sensor arrangements signals may be based on:
a measurement of a vector quantity in a direction parallel to the head-toe direction of the body of the subject; or
a measurement of a vector quantity in a direction perpendicular to the head-toe direction of the body of the subject; or
a combination of multiple vector quantities in different directions.

Thus, one-axis, two-axis or three-axis measurements, such as accelerometer measurements, may be used. Furthermore the sensor arrangement may comprise an array of sensors, for example a 2D array of sensors forming a pressure mat. In such a case, there may be a 2D signal that changes over time.

The fluid accumulation for example comprises internal bleeding.

The invention also provides a method for detecting fluid accumulation in a body region of a subject, comprising:
obtaining force, motion or pressure signals, induced by the beating of the heart of the subject, wherein the signal varies in dependence on the density distribution of the body region; and
analyzing changes in the signals over time thereby to determine that fluid accumulation has taken place based on detecting changes in said density distribution.

The invention also provides a computer program comprising computer program code means which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows the relationship between an ECG signal and a BCG signal;
Figure 2 shows a system in accordance with the invention for detecting fluid accumulation in a subject;
Figure 3 shows raw accelerometer signals for a three-axis accelerometer over time;
Figure 4 shows the output of the EMD process, known as IMFs;
Figure 5 shows the Fourier Transform amplitudes for the IMFs of Figure 4;
Figure 6 shows an experimental setup with a pressure mat, ECG electrodes, a respiration measurement band and three-axis accelerometers;
Figure 7 shows two different features calculated from the measurement signal;
Figure 8 shows changes in signal characteristics using various features calculated from the short-time based analysis of the raw signal;
Figure 9 shows the variations in energy for three different accelerometer locations;
Figure 10 shows results for a weight measurement approach based on force sensing;
Figure 11 shows a trend line for the IMF2 energy for the subject as a function of volume intake and the two plots normalized to the baseline; and
Figure 12 shows a method for determining that fluid accumulation has taken place.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for detecting fluid accumulation in a subject. A movement induced by the beating of the heart is analyzed over time, by monitoring a movement, pressure or force. Changes in density caused by fluid accumulation result in changes in the sensor arrangement signals. Changes are used to indicate that fluid accumulation such as internal bleeding is likely to have taken place.

The invention makes use of analysis of movement of the body resulting from the beating of the heart.

A ballistocardiogram (BCG) is defined as the reaction (displacement, velocity or acceleration) of the body to cardiac ejection of blood. Consequently, it is an integration of multiple forces related to movements of blood inside the heart, inside the arteries (primarily the aorta), and movement of the heart itself. Thus, this invention makes use of analysis of a signal similar to a ballistocardiogram. Whereas this type of signal is conventionally used for heart rate or respiration measurement, with the sensor placed as close to the heart as possible, the invention enables this type of motion signal to be detected remote from the heart to enable the propagation of the heart pressure wave to be detected through regions of interest of the body.

The ballistocardiogram (and the signal picked up by motion, force or pressure sensing in accordance with the invention) is inherently a multi-dimensional signal, which can be measured at all directions and all sides. These signals are strongest at the head-to-toe direction hence most BCG measurement techniques focus on the longitudinal, head-to-toe component.

It is known to use the BCG signals, in particular when implementing SCG (seismocardiography), to measure the heart of a patient and the sensing electrodes are placed as close to the heart as possible. In addition, the respiration rate can be derived from the system. The latter has the largest force in the dorso-ventral direction. However, BCG signals can be measured more generally at different sides, and at the places distant from the heart. Also they can be measured by placing the sensors on objects that the subject is touching, such as a bed, or a back support, or chair.

The invention is based on the observation that motion/pressure signal such as BCG signals have characteristics which change as a result of fluid accumulation. This happens because the movement, or vibration, or resonance of the body parts resulting from the forces generated by the beating heart and travelling blood change as a result of the fluid accumulation.

In other words, normally the internal organs vibrate in a particular manner as a result of the beating heart, but this changes because fluid is being accumulated, and their vibration characteristics change (for example damping and stiffness), which also changes the BCG signal that is measured externally to the body.

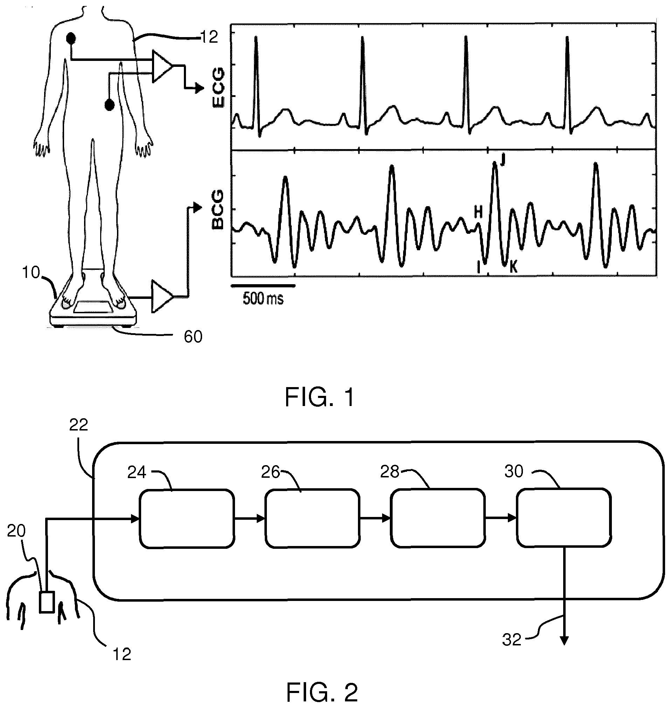

Figure 1 shows the relationship between an ECG signal and a BCG signal as measured by a pressure mat 10 on which a subject 12 is standing.

The invention is based on the use of BCG-type information to measure internal fluid accumulation such as internal bleeding. Any suitable methods may be used to detect the movement of the body and thereby obtain the BCG-type signal. Examples are accelerometers, gyroscopes, force sensors and pressure sensors. A weighting scale for example uses load cells.

Accelerometers are already widely used in body sensors, for example in respiration sensors. Pressure sensors or load cells can be integrated in mats that can be placed in contact with a subject, for example underneath a subject. It is also known to measure BCG signals using cameras, and such systems may also be used. Cameras provide a non-contact sensing approach. Motion sensors may also be used to implement non-contact sensing, by monitoring movement of objects in contact with the subject. Such an object may be a suspended bed or a bed positioned on flexible legs, or a seat with a flexible back support. There does not need to be direct contact for example if there is a mattress between. Thus, more generally, monitoring movement of objects whose motion is caused by the BCG is possible.

In such cases, the sensor arrangement is not located on the body of the subject but is nevertheless for sensing from a known area of the body even though the sensing is contactless.

Thus, the invention is applicable for different types of sensors.

Figure 2 shows a system in accordance with the invention for detecting fluid accumulation such as internal bleeding in a subject 12, The system comprises a sensor arrangement 20 for obtaining a sensor signal in respect of movement the subject. For convenience, the signal will from this point onwards be referred to simply as a ballistocardiography signal. The sensor arrangement 20 for example comprises an accelerometer or a pressure sensor or a combination of these, for measuring an oscillation of the body.

A processor performs analysis of the sensor arrangement signals. It may simply analyze the raw sensor arrangement signals over time for example using a machine learning or deep learning algorithm. However, to show the underlying concepts of the invention more clearly, a processing approach based on more identifiable and structured signal analysis will be described.

For this example, the processor 22 comprises:
a first signal processing unit 24 for signal pre-processing. This for example comprises filtering and decomposition enabling noise removal and extraction of the signal components;
a feature extraction unit 26 to isolate a feature of interest of the ballistocardiography signal;
a feature analysis unit 28 to determine a parameter of the feature of interest of the ballistocardiography signal. This feature of interest is in particular an energy or signal strength related feature;
an output unit 30 to determine from changes in the determined parameter that internal bleeding has taken place. This may be used to generate an alert 32 for indicating that internal bleeding has taken place.

As discussed above, organs within the body will experience oscillatory forces due to the beating of the heart, blood flow inside the large arteries (in particular the aorta) and due to breathing. To capture these oscillations, movement of the body of the patient can be measured, which will consist of movement of the patient, heart rate components and breathing components (i.e. oscillatory forces).

The beating of the heart and the movement of the torso (breathing) will induce a force that travels through the body. Any compartment (i.e. organs, tissue, fluids) that is inside the body will react differently depending on the nature of that compartment, such as the density and size, and also the damping and stiffness characteristics. When looking at the frequency domains of captured oscillatory signals, the lower frequencies originate from movement of the subject and the breathing of the patient (≈0.4Hz), the heart rate is around 1Hz and the frequency components above the measured heart rate are mainly the oscillations of the body due to the force exerted by the beating of the heart.

The concept is based on detecting the changes in (local) body density as a result of fluid accumulation such as internal hemorrhage. The body density can in fact change due to various reasons, such as:
accumulation of urine in the bladder;
blood leakage caused by sepsis;
consumption of food;
consumption or addition of liquids (e.g., medication or intravenous fluids);
internal hemorrhage;
inflammation.

The body density changes have different time frames and different impacts on the oscillations. For example, the consumption of food will generate a large and sudden change. This sudden change can be filtered out of the system by correcting the baseline, essentially implementing high pass filtering to remove the low frequency components.

The addition of fluids via intravenous drip is a gradual process that is redistributed across the body via the blood stream (so no accumulation of fluid) and inflammation is a relatively slow process that will change the local density of the tissue (i.e. higher perfusion, swelling). The consumption of solid food (i.e. with low water content) can also be clearly differentiated from bleeding or liquid consumption because the changes are in opposite direction.

The different processing steps used in this particular example will now be discussed in more detail.

### First signal processing

The first signal processing unit 24 implements analysis windows of a predetermined duration, such as 5 to 60 seconds, for example 30 seconds. This predetermined duration covers a plurality of heart cycles. The windows collect sufficient data for the signal properties of interest to be measured.

These windows are analyzed for an overall analysis period, for example at least two minutes, such as 5 minutes. The raw sensor data, such as one or more accelerometer signals, in a time interval [t1 t2] is thus divided into analysis periods of 30 seconds such as [t1 t1+30], [t1+1 t1+31], and so on. The overall time period is sufficient for the fluid accumulation level to have changed.

The analysis periods may also have a partial overlap, for example windows that overlap by 50%.

### Feature extraction

The feature extraction unit 26 computes features for the signals windowed by the first signal processor. In one example, the computation comprises decomposing the original signal into its constituent frequency components. Adding these frequency components together will lead to the original signal. Since the frequency of breathing and the heart beat are significantly different, frequency decomposition leads to frequency features corresponding solely to breathing and heart beat along with other features corresponding to the oscillations due to action of the beating heart (as discussed above).

For frequency decomposition, Empirical Mode Decomposition (EMD) may be used, but alternatively wavelet transforms and filter-bank analysis can also be used. The advantage of the EMD, over these two methods is that EMD is non-parametric.

The resulting decomposition signals using EMD are called intrinsic mode functions (IMFs).

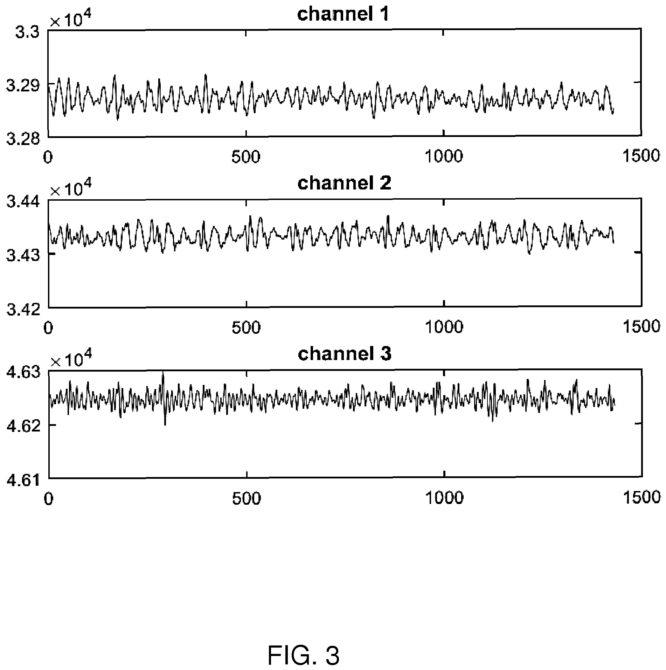

By way of example, Figure 3 shows raw accelerometer signals for a three-axis accelerometer over time.

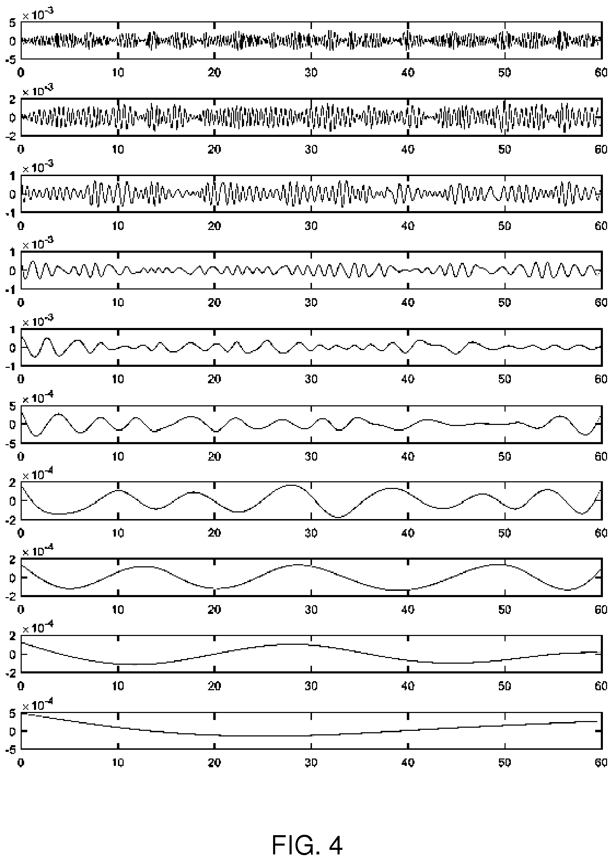

The raw signals are normalized to have zero mean, by dividing the signal by its mean and subtracting one, before applying EMD. The output of the EMD process are the IMFs, shown in Figure 4.

These IMFs are time domain signals, but divided into different frequency bands, i.e. the x-axis shows time in seconds.

The number of the IMFs is dependent on the raw signal. IMFs have decreasing frequency content, the first IMF (at the top of Figure 4) models the high frequency noise and the last IMF (at the bottom of Figure 4) models the DC of the raw signal.

Processing of short time windows (e.g. 60 seconds) is used to calculate the IMFs. The windows are shifted in time (with a shift amount of the order of seconds, but less than the window length). Window lengths varying from 10 seconds to 60 seconds may for example be used, with comparable results. The optimal window length depends on the frequency being measured, with a longer window more suited to low frequencies and shorter windows for high frequencies.

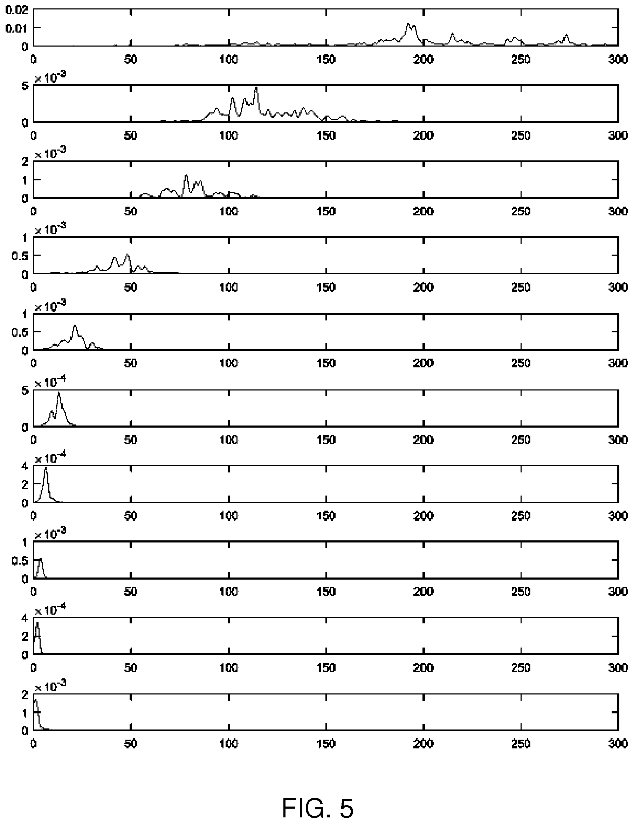

Figure 5 shows the Fourier Transform amplitudes for the IMFs of Figure 4.

These Fourier Transform amplitudes are frequency domain signals, i.e. the x-axis shows frequency in Hz.

As it can be seen from Figure 5, the first two IMFs contain high frequency components, and these are the components of interest for this application. The third (in this particular example) and later components contain the heart rate.

The frequency content of the IMFs is ordered from high frequencies to low frequencies, in other words first IMF contains the highest frequencies, and the frequency decreases as IMF number increases. The heart rate is for example visible in the third component, but that depends on the signal characteristics.

The breathing rate can be also measured. For this example, it is observed in the 6th component. Since components above the heart rate are of interest, the heart rate itself can be used to extract the right features.

As indicated above, the processing is not very sensitive to the exact choice of a cut-off frequency. This means that all components higher than 1.67 Hz or above a determined heart frequency of the subject may be used. In noisier conditions, it may be preferable to focus on selected frequency bands, and to calculate features for these bands.

### Feature analysis

The feature analysis unit 28 is used for analyzing the decomposition signals with frequencies higher than the heart rate, in order to extract representative features, such as energy. Especially the energy feature, which is linked to the signal amplitude, is associated with fluid accumulation. The energy and/or Hilbert transformation may be used to measure and study the changes in the oscillations over time.

For each one of the IMFs of Figure 4, the energy component of the normalized time domain signal is obtained by applying an 'Energy' measure or 'Hilbert' transform.

The energy measure is the root mean squared energy of the (normalized) signal. The signal is for example normalized to have zero mean before calculating the energy. In this case (i.e. after the normalization) the energy measure is similar to the standard deviation of the signal. However, normalization of the signal before calculating the energy, is not strictly necessary.

An alternative measure, also relating to the energy content, is the Hilbert transformation applied to the chosen IMF signal.

For the calculation of the Hilbert feature, the Hilbert transform (HT) is used for local envelope detection. The HT feature is defined as the mean value of the modulus of the complex analytical signal obtained from the window under consideration. The analytical signal is defined as the sum of the complex signal, comprising the original signal and its Hilbert transform.

The Hilbert transform is essentially a transformation that makes it easy to track the envelope of a signal. The envelope is representative of variations in amplitude and thus of energy of the signal.

These features are calculated for the IMFs that have higher frequencies than the heart rate (either based on the heart rate itself, or based on a threshold level).

### Output unit

The output unit 30 implements estimation of a trend line fitting the features identified by the feature analysis. A running average is made of these features to detect any (gradual) changes in features resulting from changes inside the body. Based on the slope of the trend line, an alert may be generated (e.g., if the slope is negative an alert may be generated for potential internal bleeding).

As mentioned above, the feature analysis may focus on frequency bands higher than the resting heart rate. Experimentally, details of which are given below, it has been shown that these frequency bands are more sensitive to local changes in body density and thus can provide better distinction. An exact lower cut-off frequency is not of critical importance, as long as the frequencies lower than the heart rate are not the only ones that are included. For example, focusing on frequencies in a band is suitable with a lower threshold above the heart rate, for example of 1.67Hz or above (for a fixed lower threshold) or based on the actual heart rate.

For the frequency domain analysis methods, such as using filter-banks, a cut-off frequency is set (e.g. in the range 1.67Hz to 5Hz). If the measurement of the exact heart rate is available, it can be used as an input to derive a better cut-off frequency.

The filtering is not essential. Instead, it is simply required that the higher frequencies are included in the analysis. However the detection is also possible if both higher and lower frequencies are also included, for example as is the case for raw signal processing without frequency decomposition.

Thus, there are many ways that the signal can be processed. One options is to only use frequencies higher than the heart rate and another option is to use all frequencies, also including heart rate.

### Frequency domain vs. time domain

The example above is based on time domain analysis, in order to derive the IMF signals of Figure 4. The decomposition may however be performed in the frequency domain.

A time domain analysis is also possible without frequency decomposition.

For example, an energy feature may be calculated directly from the raw signal, without decomposing the raw signal into components of different frequencies. In low noise conditions, where the patient is static, this simpler method is suitable. The observed effect is present even without excluding the heart rate signal. Excluding the heart rate signal, makes the differentiation between conditions clearer, but may not be necessary.

The time domain method for example comprises the following steps:
A window period for the analysis is defined. For example, a running average window of 20 seconds may be used with calculations every second (giving a 95% window overlap). These windows are analyzed for an overall analysis period, such as 5 minutes.

For an accelerometer, one or more axes of the accelerometer may be chosen for analysis. If multiple axes are chosen, the data of the axes can be added to combine them.

The energy measure (e.g. square root of the sum of squares of each data point) is calculated, or else the Hilbert Transform of the signal may be used. Filtering or transformation of the signal may optionally be considered prior to calculation of features.

The example above is based on the decomposition of the signal into time domain signals with different frequency content and the calculation of features from one or more of the decomposed signals. These features are used to distinguish between fluid accumulation or no fluid accumulation.

The decomposition step is not always necessary. For some subjects, the difference between the conditions can be observed also without decomposing the signal into frequency bands. The method is however typically more robust with the decomposition.

Thus, in the preferred implementation, the second IMF is selected, and features are calculated from that signal. A more adaptive approach is to first determine if decomposition is needed, and if needed which frequency bands should be used. In other words, an adaptive processing method may be used, in which depending on the signal characteristics, the features are calculated from the whole signal, or from a selected set of (one or more) decomposition signals.

For both the frequency domain and time domain methods, it can be preferable to select the specific accelerometer axes, and to calculate features only for these axes. One or more of the axes can be used to reduce the noise on the signal. For example, the motion and/or external vibration of the subject can be derived from one axis and used to correct the signal along the axis capturing the head-to-toe direction. Another option is to select the channel with the highest ratio of high frequency signal energy to lower frequency signal energy.

Since the oscillations originating from the beating heart are mainly in the direction parallel to the body (i.e. from head to toes), axes in the same directions are more suitable for detecting changes. Thus, in a preferred embodiment the axis aligned with the body is selected and the energy features extracted from that signal are used.

However, in certain cases, using all accelerometer axes (i.e. summing them up) is also possible.

### Experimental results

Experiments have been performed to show that fluid accumulations in the body can be measured using this BCG based approach, in particular based on accelerometers and pressure sensors.

A water intake was used in the experiments to mimic fluid accumulation in the body. In this case, the accumulation of fluid is self-evidently in the stomach and small intestine, so the sensor arrangement is located below the stomach and small intestine, for example around the lower waist or hips. A measurement time of around 5 minutes was used, with the subject remaining still (upright or lying flat).

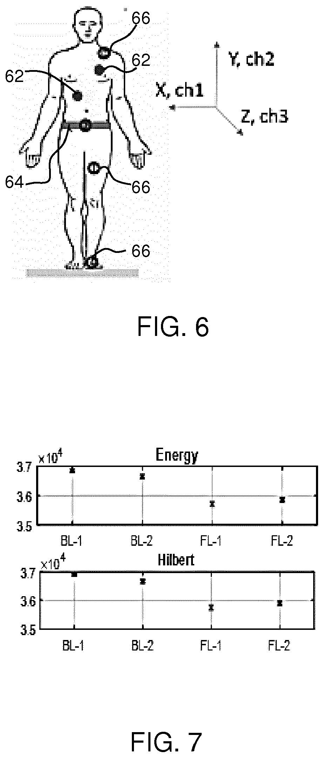

Figure 6 shows the experimental setup with a pressure mat 60, ECG electrodes 62, a respiration measurement band 64 and three-axis accelerometers 66. In the workflow, a baseline was measured followed by an intake of water (of different volumes) and a subsequent measurement. The measurements were performed in both standing and lying positions.

The results based on the use of accelerometers are first discussed. They provide a more clinically applicable situation were the subject is supine. However, similar results may be obtained using weight scale measurements.

The acceleration signal in a direction parallel to the body was used.

Figure 7 shows two different features calculated from the signal. Each feature is shown for two baseline measurements BL1,BL2 and two fluid accumulation conditions FL1 and FL2. These are signals for the same subject, in the lying position only, with repeated measurements 1 and 2.

Two different energy measuring features are calculated from the signal.

Energy is the energy measure for the whole signal in this example. i.e. without frequency decomposition.

Hilbert represents a measure of signal energy, based on calculating the Hilbert transformation of the whole signal.

Both the energy measure and the Hilbert transformation give a good distinction between the baseline (BL) and the corresponding fluid accumulation (FL) conditions. In other words, the variability of the signal monitored over time changes significantly depending on the fluid inside the body.

Due to this significant change, which is observable even for a small amount (300 ml) of accumulated fluid, changes in the signal energy or signal strength are monitored and used to generate alerts for fluid accumulation (water in the stomach and small intestine in this example, but used to validate the possibility of detecting internal bleeding).

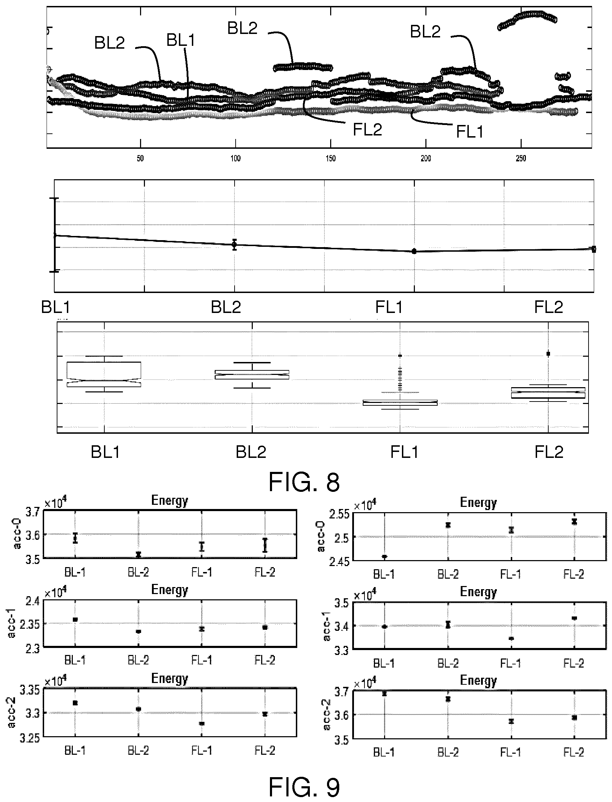

Figure 8 shows changes in signal characteristics (in particular the energy measure) using various features calculated from the short-time based analysis of the raw signal.

The top plot shows the mean energy feature per processing window (60 seconds) for the total measurement duration of 5 minutes. The middle plot shows the mean (the line) and standard deviation (the error bars) of the energy feature for the four test conditions (BL1, BL2, FL1, FL2), and the bottom plot shows a box-plot of the energy feature for the four test conditions. The reduction of the energy as a result of fluid accumulation is clearly visible and quantifiable from these results.

As expected, when using the heart as the source of the oscillation the position of the accelerometer has an impact on the signal.

Figure 9 shows the variations in energy for three different accelerometer locations: acc-0 (left shoulder), acc-1 (left thigh) and acc-2 (stomach). In general, accelerometer locations between the heart and toes are particularly suitable positions based on the origin and propagation of BCG signals. The positions below the heart give the best results for this particular case, because the fluid is in the stomach. Of course for fluid accumulation above the heart, this would not be the case.

The left column is for one subject and the right column is for another subject. For each subject, BL1 and BL2 are repeated baseline measurements and FL1 and FL2 are repeated measurements for the fluid accumulation condition. They are all for the subject lying down.

Figure 9 shows the robustness of the signal, since the measurements were conducted at different times. Note that the calibration of the accelerometers (i.e. vector analysis) and positioning will have an impact on the signal quality.

Experiments have also been performed to measure the sensitivity when using a pressure sensor while the subject is in a standing position. Measurements performed on the same subject at the same time show that signals characteristics of the pressure sensor signals are comparable to the signal characteristics of the accelerometer signals. Thus, similar sensitivity measures are applicable for accelerometer signals as for pressure signals.

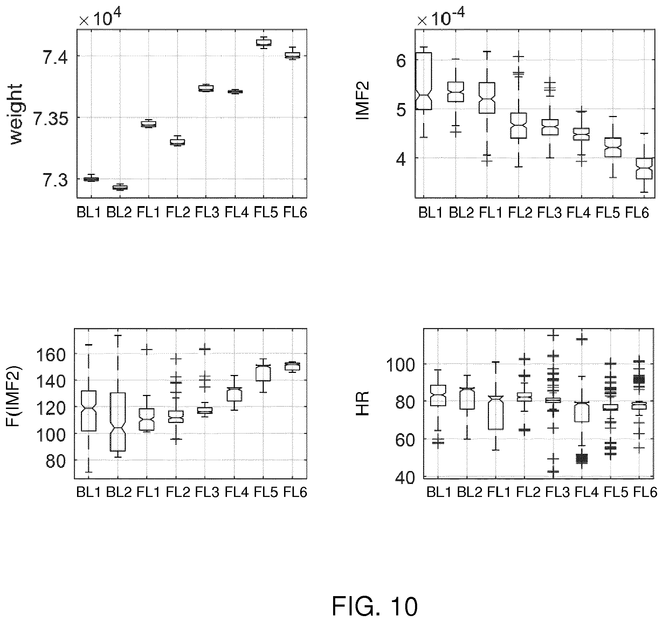

Figure 10 shows the results of signal analysis based on force measurements obtained by load cells of weight scale.

Load cells sensors in a weight scale were used to measure the BCG signal, with fluid accumulation increased by 300ml of water. BL1 and BL2 represent two repeated baseline measurements for the subject. FL1 and FL2 are repeated measurements for the subject following a first consumption of 300ml of water. FL3 and FL4 are a second set of two repeated measurements following a second consumption of 300ml of water. FL5 and FL6 are a third set of repeated two measurements following a third consumption of 300ml of water. As can be seen from Figure 10, the weight (top left image) increases with water intake and the energy of the IMF2 (top right plot) shows a decreasing trend.

The bottom left plot shows the frequency of the IMF2 signal (x 60, i.e. in cycles per minute), and the bottom right plot shows an estimated heart rate.

The results of the two measurements show similar decrease of energy of the IMF2 signal. Even with a limited amount of repeats, the 300ml of fluid accumulation is significantly different from the baseline.

The energy of the IMF2 signal may thus be used to create a trend line, wherein the trend is used to provide an alert that there is internal bleeding.

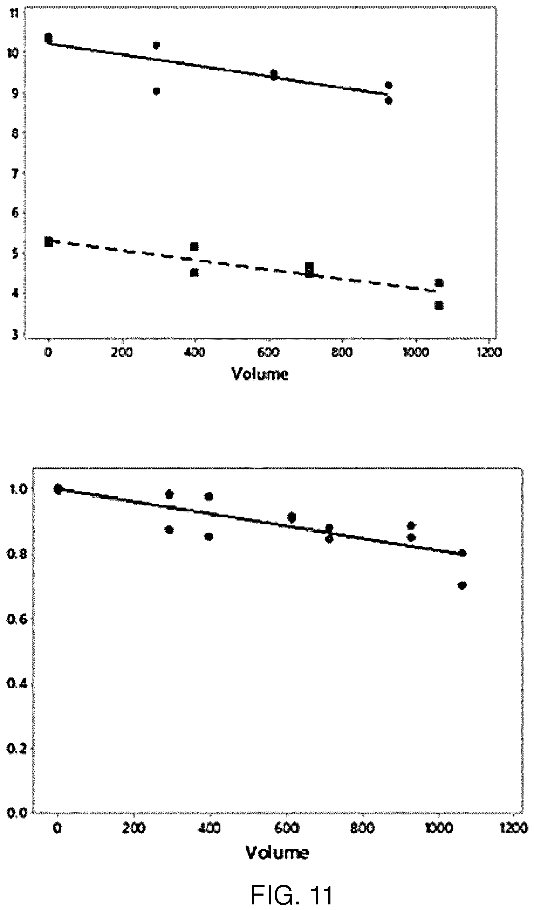

The top image of Figure 11 shows, as a solid line, a trend line for the IMF2 energy for the subject represented in Figure 10 as a function of volume intake.

The dotted line shows data for another subject (the underlying data for which is not shown). When normalizing to the baseline (volume = 0) the two data sources show a good correlation with the volume intake as shown in the bottom image of Figure 11 (which shows both plots of the top image but overlapped due to the baseline correction).

A gradient of the trend line may be used as the trigger for an alert, for example if the gradient exceeds a certain threshold (i.e. is less than a particular negative threshold value).

An amplitude difference between the baseline and the measured condition above a threshold may also be used as a trigger. In practice, this could accomplished by normalization using the baseline reading. Any deviation greater than x% (such as 2.5%) could be used to trigger an alert.

The appropriate trigger depends on the detection method. For a general feature based detection, analysis of the patterns of change or trends can be used.

For a specific feature based method, where a specific feature is correlated with the fluid accumulation in the body, a threshold based method can be used. In other words, once this feature shows particular values an alert can be generated.

For a data based model, where a machine learning approach (also including deep learning) is applied, the probability at the output of the classifier will be observed and used to make a decision to trigger an alert or not.

For a time series based approach, looking for a trend in the signal characteristics would be preferred.

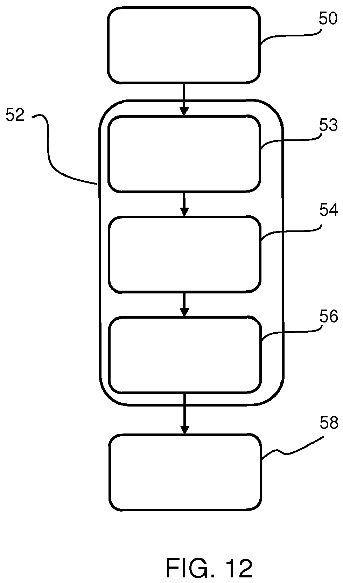

Figure 12 shows a method for detecting fluid accumulation in a subject, comprising:
in step 50, obtaining force, motion or pressure signals, induced by the beating of the heart of the subject, obtained from a location on an opposite side of the body region to the heart of the subject; and
in step 52, analyzing changes in the signals over time thereby to determine that fluid accumulation has taken place.

For the specific example above, the step 52 can be broken down to the steps of:
performing feature extraction in step 53 to isolate a feature of interest of the ballistocardiography signal;
determining in step 54 a parameter of the feature of interest of the ballistocardiography signal over a time period of multiple cardiac cycles; and
in step 56 determining from changes in the determined parameter that fluid accumulation has taken place.

An alert is then provided in step 58.

As discussed above, the system makes use of a processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

A time-series based processing method is discussed above, but it is also mentioned that machine learning may be used. A machine learning classifier would continuously generate a probabilistic score as to whether fluid accumulation is detected or not. Deep learning based methods may also be used.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for detecting fluid accumulation in a body region of a subject, comprising:
a sensor arrangement (20) for obtaining a force, motion or pressure signal, induced by the beating of the heart of the subject, wherein the signal varies in dependence on the density distribution of the body region; and
a processor (22), which is adapted to analyze changes in the sensor arrangement signals over time thereby to determine that fluid accumulation has taken place based on detecting changes in said density distribution.

2. The system as claimed in claim 1, wherein the sensor arrangement is:
for location on an opposite side of the body region to the heart of the subject; or
for sensing from an opposite side of the body region to the heart of the subject; or
for sensing movement of an object coupled to the subject in such a way that movement of the object is induced by the beating of the heart.

3. The system as claimed in claim 1 or 2, wherein the sensor arrangement (20) comprises:
one or more pressure sensors; and/or
one or more inertial sensors.

4. The system as claimed in any one of claims 1 to 3, wherein the sensor arrangement comprises:
a sensor arrangement for wearing on the head;
a sensor arrangement for wearing around the waist;
a sensor arrangement for wearing on the back;
a sensor arrangement for wearing on the leg or feet;
a sensor arrangement on which the subject is to stand, sit or lie;
a non-contact sensor.

5. The system as claimed in any one of claims 1 to 4, wherein the processor (22) is adapted to:
perform feature extraction to isolate a feature of interest of the ballistocardiography signal;
determine a parameter of the feature of interest of the sensor arrangement signals; and
determine from changes in the determined parameter that fluid accumulation has taken place.

6. The system as claimed in claim 5, wherein the parameter comprises a measure of signal energy, signal strength or signal variance.

7. The system as claimed in claim 5 or 6, wherein the processor comprises a decomposition algorithm for decomposing the ballistocardiography signal into frequency components, wherein the feature of interest comprises a frequency component in a frequency band which lies above a fixed lower threshold or above a determined heart rate frequency of the subject.

8. The system as claimed in any one of claims 5 to 7, wherein:
the parameter comprises a Hilbert transform of the extracted feature, and the processor comprises a Hilbert transformation unit for implementing the Hilbert transform; or
the parameter comprises an energy, strength, variance or amplitude measurement of the extracted feature.

9. The system as claimed in any one of claims 5 or 6, wherein:
the parameter comprises a Hilbert transform of the raw sensor arrangement signals and the system comprises a Hilbert transformation unit for implementing the Hilbert transform; or
the parameter comprises an energy, strength, variance or amplitude measurement of the raw sensor arrangement signals.

10. The system as claimed in any one of claims 1 to 4, wherein the processor is adapted to apply a machine learning or deep learning algorithm to determine that fluid accumulation has taken place.

11. The system as claimed in any of claims 1 to 10, wherein the processor is adapted to analyze changes in the sensor arrangement signals over a time frame of at least two minutes.

12. The system as claimed in any one of claims 1 to 11, wherein the sensor arrangement signals are based on:
a measurement of a vector quantity or set of vector quantities in a direction parallel to the head-toe direction of the body of the subject;
a measurement of a vector quantity or set of vector quantities in a direction perpendicular to the head-toe direction of the body of the subject; or
a combination of multiple vector quantities in different directions.

13. The system as claimed in any one of claims 1 to 12, wherein the fluid accumulation comprises internal bleeding.

14. A method for detecting fluid accumulation in a body region of a subject, comprising:
obtaining force, motion or pressure signals, induced by the beating of the heart of the subject, wherein the signal varies in dependence on the density distribution of the body region; and
analyzing changes in the signals over time thereby to determine that fluid accumulation has taken place based on detecting changes in said density distribution.

15. A computer program comprising computer program code means which is adapted, when said program is run on a computer, to implement the method of claim 14.
